(19)

Europäisches Patentamt
European Patent Office
Office européen des brevets

(11) **EP 3 815 723 A1**

(12) **EUROPEAN PATENT APPLICATION**

(43) Date of publication:
**05.05.2021 Bulletin 2021/18**

(51) Int Cl.:
**A61M 1/10** (2006.01)     **A61M 1/12** (2006.01)

(21) Application number: **19206637.1**

(22) Date of filing: **31.10.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Galway-Mayo Institute of Technology H91 T8NW Galway (IE)**

(72) Inventors:
 • **Morris, William Gerard**
   **Athenry, Co. Galway (IE)**
 • **Delassus, Patrick**
   **Kilcolgan, Co. Galway (IE)**

 • **Stefanov, Florian**
   **Oranmore, Co. Galway H91 RDH9 (IE)**
 • **White, Sharon**
   **Enniscorthy, Co. Wexford (IE)**
 • **McCarthy, Eugene**
   **Moycullen, Co. Galway H91 XKC1 (IE)**
 • **Veerasingam, David**
   **Athenry, Co. Galway (IE)**
 • **Thompson, John**
   **Dublin, 13 (IE)**
 • **O'Halloran, Tony**
   **Turloughmore, Co. Galway H65 YK26 (IE)**

(74) Representative: **Purdylucey Intellectual Property**
   **6-7 Harcourt Terrace**
   **D02 FH73 Dublin 2 (IE)**

(54) **A DEVICE TO REDUCE LEFT VENTRICULAR AFTERLOAD**

(57)     A device to reduce left ventricle afterload in a target blood vessel in a mammal comprises a cushion (2, 22) configured for positioning within a lumen of the target blood vessel (3) and receipt of a fluid (15) and configured to contract during systole and expand during diastole, a reservoir (4, 24) having a cavity for receiving the fluid (15), and a conduit (5) extending between and fluidically coupling the cushion and the reservoir, whereby during use fluid in the cushion is transferred to the reservoir during systole and returned to the cushion during diastole. The cushion (2, 22) has an annular cross section defining a central lumen (8) for blood flow and is configured for positioning in the lumen of the target vessel abutting an inner wall of the target vessel, whereby during use blood flow is directed through the central lumen of the cushion.

Fig. 1A

Fig. 1B

EP 3 815 723 A1

## Description

Field of the Invention

The present invention relates to a device to reduce left ventricle afterload.

Background to the Invention

Chronic HF affects millions worldwide and the existing treatments have failed to address this debilitating condition. In the majority of patients, the arteries, and the aorta in particular, can become stiffened, losing its ability to dampen the pressure wave generated each time the left ventricle contracts. Instead the pressure wave moves quickly down the aorta and is partially reflected back towards the heart at a faster rate than normal. This early reflected waveform returns to the heart during systole when the ventricle is still ejecting blood, thus increasing the systolic duration, elevating LV afterload, ultimately leading to LV hypertrophy and increased myocardial oxygen demand. Simultaneously, this increase in afterload decreases stroke volume and reduces diastole, thus compromising coronary perfusion. Reflective wave magnitude has been shown to be highly correlated with rehospitalisation and death in HF patients.

The treatment of heart failure is currently dominated by pharmaceutical companies, however there are a number of devices in various stages of development. The most prominent of these new technologies are cardiac resynchronization therapy device, ventricular assist devices and cardiac contractility modulation.

The goals of pharmacologic therapy of heart failure are to improve symptoms (including risk of hospitalisation), slow or reverse deterioration in myocardial function, and reduce mortality. Improvement in symptoms can be achieved by diuretics, beta blockers, ACE inhibitors, ARBs, ARNI, hydralazine plus nitrate, digoxin, and aldosterone antagonists. Prolongation of patient survival has been documented with beta blockers, ACE inhibitors, ARNI, hydralazine plus nitrate, and aldosterone antagonists. More limited evidence of survival benefit is available for diuretic therapy. Despite the myriad of pharmaceutical options available, mortality is comparable to that of the most common cancers, with <50% of patient dying within 4 years of diagnosis survival.

Gene therapy approaches to heart failure (HF) are under investigation but have not yet been shown to be of clinical value. Studies of gene therapy for HF are based upon advances in gene transfer technology, including development of safe and efficient vectors and delivery methods as well as identification of appropriate therapeutic targets.

Limited data are available on the efficacy of enhanced external counterpulsation (EECP) in patients with chronic HF. EECP is a technique that increases arterial blood pressure and retrograde aortic blood flow during diastole (diastolic augmentation). Cuffs are wrapped around the patient's legs and, using compressed air, sequential pressure (300 mmHg) is applied (from the lower legs to lower and upper thighs) in early diastole to propel blood back to the heart.

Cardiac resynchronization therapy (CRT) involves simultaneous pacing of both ventricles (biventricular or BiV pacing) or of one ventricle in patients with bundle branch block to reduce dyssynchrony. Resynchronization may improve pump performance, reduce functional mitral regurgitation, and reverse the deleterious process of ventricular remodeling in patients with heart failure (HF). Most patients who satisfy criteria for CRT implantation are also candidates for an ICD and receive a combined device. An implantable cardioverter-defibrillator (ICD) for primary or secondary prevention of sudden cardiac death. Despite the potential benefits only 5.6% of real-world patients met resynchronization therapy criteria in a Spanish registry.

Cardiac Contractility Modulation (CCM) is a treatment for patients with moderate to severe left ventricular systolic heart failure (NYHA class II-IV) which enhances both the strength of ventricular contraction and the heart's pumping capacity. The CCM mechanism is based on stimulation of the cardiac muscle by non-excitatory electrical signals (NES), which are delivered by a pacemaker-like device. CCM is particularly suitable for the treatment of heart failure patients with normal QRS complex duration (120ms or less) and has been demonstrated to improve the symptoms, quality of life and exercise tolerance of heart failure patients. CCM is approved for use in Europe, but not currently in North America.

Ventricular assist devices (VAD) are an expensive electromechanical circulatory device that is used to partially or completely replace the function of a failing heart. These devices are implanted in specialist centres with large multi-disciplinary support teams. Some VADs are intended for short term use, typically for patients recovering from heart attacks, while others are intended for long-term use (months to years and in some cases for life), typically for patients suffering from advanced heart failure. The majority of VADs on the market today are bulky with options for women and children, limited. Bleeding is the most common postoperative early complication after implantation or explantation of LVADs, necessitating reoperation in up to 60% of recipients. The implications of massive blood transfusions are great and include infection, pulmonary insufficiency, increased costs, right heart failure, allosensitization, and viral transmission, some of which can prove fatal or preclude transplantation. Treatment of VAD-related infection is exceedingly difficult, and many patients die of infection despite optimal treatment. Considering the multitude of risks and lifestyle modifications associated with ventricular assist device implant, it is important for prospective patients to be informed prior to decision making.

US9017359 and US9333328 describe a device and method for treating pulmonary arterial hypertension by improving compliance in the pulmonary vasculature. The device employs a compliant cushion disposed in the low-pressure pulmonary vasculature and containing a fluid, a compliant reservoir for fluid disposed at a distance from the compliant cushion, and a long conduit extending between and fluidically coupling the compliant cushion and reservoir. The long transvascular conduit results in a large pressure drop along the length thus affecting performance. In addition, for the device to work in a high-pressure system such as an aorta, the compliant cushion needs to be large, resulting in partial blockage of the vessel.

It is an object of the invention to overcome at least one of the above-referenced problems.

Summary of the Invention

The Applicant has addressed the problems of the prior art, by providing a device with a cushion provided in the form of an annular sleeve with a central conduit for blood flow. The cushion is configured for positioning in the lumen of the vascular system abutting an inner wall of the vascular system such that blood flow is channelled through the central lumen. The provision of the cushion as an annular sleeve is a more efficient way of increasing the area of the blood/cushion interface while maintaining a large conduit through the centre of the sleeve for blood flow through the vessel. In addition, providing the cushion as a sleeve allows blood to travel through the cushion rather than around it, thus eliminating the risks caused by impacting of blood against the cushion.

In a first aspect, the invention provides a device (for example a device to reduce left ventricle afterload in a target blood vessel in a mammal). The device comprises a cushion configured for positioning within a lumen of a target blood vessel and receipt of a fluid and configured to contract during systole and expand during diastole, a reservoir having a cavity for receiving the fluid, and a conduit extending between and fluidically coupling the cushion and the reservoir. The device is configured such that during use fluid in the cushion is transferred to the reservoir during systole and returned to the cushion during diastole. The cushion preferably has an annular cross section defining a central lumen for blood flow and is configured for positioning in the lumen of the target vessel abutting an inner wall of the target vessel, whereby during use blood flow is directed through the central lumen of the cushion. The cross-section of the annular cushion is generally configured to match the cross-section of the target vessel into which the device is to be implanted, and may be circular, oval-shaped, elliptical or have a regular or irregular polygonal shape. The cushion may be straight or curved, or tapered along all or part of its length.

In one embodiment, the cushion is a compliant cushion. The compliant cushion is typically configured for compression by a pressure wave in blood passing through the central lumen of the cushion during systole to push fluid to the reservoir and decompression during diastole to actively pull fluid back into the cushion from the reservoir.

In one embodiment, the cushion has a compliance of 5 to 200 x $10^{-4}$/mmHg, as determined by the method described below.

In one embodiment, the reservoir is configured to passively accept fluid during compression of the compliant cushion and passively discharge fluid during decompression of the compliant cushion.

In one embodiment, the device comprises an anchoring element associated with at least one end (and preferably both ends) of the cushion. The anchoring element is typically adjustable from a radially contracted configuration to a radially expanded configuration, whereby when the cushion is disposed within the lumen of the target blood vessel, the expanded anchoring element urges the end of the cushion into circumferential contact with the wall of the target blood vessel to prevent blood flow between the annular cushion and the vasculature.

In one embodiment, the anchoring element is a radially expandable stent associated with an inner circumference of one or both ends of the cushion.

In one embodiment, the conduit has a length and/or a bore of from 3mm to 50mm, more preferably about 3mm to 15mm or 3mm to 10mm.

In one embodiment, the cushion is formed from a hyperelastic elastomer with viscoelastic properties.

In one embodiment, the conduit extends outwardly (for example, radially outwardly) of the cushion, typically but not necessarily orthogonal to a longitudinal axis of the cushion. In one embodiment, the reservoir is configured for implantation into a body cavity adjacent the target blood vessel.

In one embodiment, the cushion comprises:

an outer tube configured to fit within the lumen of the target blood vessel,
an inner tube having end sections attached to an inner wall of the outer tube and a central waist section disposed between the end sections, and
a fluid receiving annular lumen defined by the outer tube and the central waist section.

In one embodiment, the cushion is a compliant cushion, and the waist section is a compliant waist section.

In one embodiment, a fluid is disposed within the annular lumen.

In one embodiment, the inner tube, outer tube (and optionally the compliant waist section) are formed from a hyper-elastic elastomer with viscoelastic properties.

In one embodiment, the reservoir for fluid comprises a structure configured for expansion without resilient deformation of the wall of the structure. One example is a foldable structure configured for adjustment from an initial folded configuration to an unfolded, fluid holding, configuration.

In one embodiment, the conduit comprises a valve and a valve actuation mechanism including remote actuation means.

In one embodiment, the reservoir for fluid comprises a compliant fluid-receiving body and the cushion is configured to passively accept fluid during diastole. In one embodiment, the reservoir has a compliance of 5 to 200 x $10^{-4}$/mmHg, as determined by the method described below.

In one embodiment, the reservoir comprises an injection port fluidically coupled to the fluid receiving cavity.

In one embodiment, the device comprises a plurality of reservoirs fluidically coupled to the cushion, for example in parallel or in series.

In one embodiment, the conduit is configured to fluidically couple with the cushion and/or the reservoir in-vivo. Various coupling means are envisaged, the details of which will be known to a person skilled in the art.

In another aspect, the invention provides a method of treating or preventing a cardiovascular condition in a subject by reducing left ventricle afterload in a target blood vessel. In one embodiment, the method comprises the steps of implanting a device according to the invention into a subject with the cushion being implanted in a lumen of the target blood vessel whereby during systole a pressure wave in the blood in the target vessel generated by the left ventricle contracts the cushion causing the fluid in the cushion to transfer to the reservoir, whereby the pressure or velocity of the pressure pulse wave is dampened.

In another aspect, the invention provides a method of treating or preventing a cardiovascular condition in a subject by reducing left ventricle afterload in a target blood vessel. In one embodiment, the method comprises the steps of implanting a device according to the invention into a lumen of the target blood vessel whereby during systole a pressure wave in the blood in the target vessel generated by the left ventricle contracts the cushion causing the fluid in the cushion to transfer to the reservoir, whereby the pressure or velocity of the pressure pulse wave is dampened.

In one embodiment, the method includes a step of delivering a volume of fluid into the device, before or after implantation. In one embodiment, 5 to 90 ml of fluid is delivered into the system. In one embodiment, when the cushion is a compliant body, the amount of fluid employed to prime the system nearly or completely fills the cushion with little or no water in the reservoir.

In one embodiment, the cardiovascular condition is selected from heart failure, hypertension, stroke, and endovascular aneurysm repair.

In one embodiment, the method is for treating or preventing heart failure, in particular a symptomatic treatment of heart failure.

In one embodiment, the reservoir is implanted in a body cavity external to, and typically adjacent, the target blood vessel.

In another embodiment, the reservoir is implanted in the target blood vessel.

In one embodiment, the cushion is implanted in arterial system. In one embodiment, the cushion is implanted in the aorta. In one embodiment, the cushion is implanted in the ascending thoracic aorta, preferably distal to the left Subclavian artery origin.

In another embodiment, the device is positioned in a distal section of the descending thoracic aorta, distal of the renal arteries.

In another embodiment, the device is positioned (or configured for positioning) in an ascending section of the aortic arch, proximal of the brachiocephalic artery.

In one embodiment, the device is positioned in the iliac artery.

In one embodiment, the device is implanted transluminally.

In one embodiment, transluminal implantation of the device comprises the steps of:

advancing the cushion to the target blood vessel percutaneously (typically over a first guidewire);
anchoring the cushion in the target blood vessel;
advancing a needle device transluminally into the cushion;
actuated the needle device to generate a hole in the cushion and the wall of the blood vessel;
advancing the reservoir percutaneously through the hole into a body cavity adjacent the target blood vessel;
advancing a radially expansible conduit percutaneously across the hole to fluidically connect the cushion and the reservoir;
advancing a catheter percutaneously into the hole in the cushion and delivering fluid into the cushion; and
plugging the hole in the cushion.

In another embodiment, the device is implanted surgically.

In one embodiment, a section of the target blood vessel into which the cushion is to be implanted is surgically excised, the cushion is implanted into a lumen of the surgically excised section of the target blood vessel, and the excised section of the target blood vessel carrying the cushion is re-connected to the target blood vessel.

In one embodiment, the method includes a step of generating a hole in a wall of the excised section of the target blood vessel, placing the conduit across the hole, and fluidically coupling the conduit with the cushion and reservoir. The cushion and conduit may be implanted into the lumen of the blood vessel and adjusted to allow the conduit project out of the hole in the blood vessel. Alternatively, the cushion may be first implanted into the lumen, and then the conduit inserted through the hole to fluidically couple with the cushion. The reservoir may then be fluidically coupled with the conduit.

In another embodiment, the device including the reservoir and conduit is implanted into the surgically excised section of the aorta.

In another aspect, the invention provides a device to reduce left ventricle afterload in a target blood vessel of a subject, comprising:

a cushion configured for positioning within a lumen of the vascular system containing a fluid and configured to contract during systole and expand during diastole;

a reservoir having a cavity for receiving the fluid; and

a conduit extending between and fluidically coupling the cushion and the reservoir, whereby fluid in the cushion is transferred to the reservoir during systole and returned to the cushion during diastole,

characterised in that the cushion is a compliant cushion configured for compression by a pressure wave in blood passing through the central lumen of the cushion during systole to push fluid to the reservoir, and decompression during diastole to pull fluid back into the cushion from the reservoir, and in which the reservoir is configured to passively accept fluid during compression of the compliant cushion and passively discharge fluid during decompression of the compliant cushion.

In another aspect, the invention provides a device to reduce left ventricle afterload in a target blood vessel of a subject, comprising:

a cushion configured for positioning within a lumen of the vascular system containing a fluid and configured to contract during systole and expand during diastole;

a reservoir having a cavity for receiving the fluid; and

a conduit extending between and fluidically coupling the cushion and the reservoir, whereby fluid in the cushion is transferred to the reservoir during systole and returned to the cushion during diastole,

characterised in that the reservoir is configured for implantation into the target blood vessel and the device is configured for fluidic coupling of the cushion and reservoir within the target blood vessel.

Other aspects and preferred embodiments of the invention are defined and described in the other claims set out below.

Brief Description of the Figures

**Figure 1A** is a longitudinal sectional view of a device of the invention with an annular compliant cushion disposed in a section of the aorta and a passive reservoir disposed adjacent the aorta, shown prior to arrival of a pressure wave in the blood, with the fluid in the device disposed within the cushion and the cushion in a relaxed configuration.

**Figure 1B** is a detail of Figure 1A, showing the anchoring stent sealing the end of the cushion to an inside of the artery.

**Figure 1C** is a detail of Figure 1A showing the inner and outer tubes of the cushion, transvascular conduit and reservoir.

**Figure 2** is a transverse sectional view taken along the lines II-II of Figure 1, showing the annular cushion filled with the fluid and the central lumen filled with blood.

**Figure 3** is an illustration of the device of Figure 1 shown during systole where the pressure wave in the blood has compressed the cushion forcing liquid into the reservoir which opens up to accommodate the fluid.

**Figure 4** is a transverse sectional view taken along the lines IV-IV of Figure 3, showing the annular cushion in a compressed configuration and the fluid transferred to the reservoir.

**Figure 5** is a longitudinal sectional view of a device according to an alternative embodiment of the invention with an annular collapsible cushion disposed in a section of the aorta and a compliant reservoir disposed adjacent the aorta, shown prior to arrival of a pressure wave in the blood, with the fluid in the device disposed within the cushion and the inner wall of the cushion in a collapsed configuration

**Figure 6** is a transverse sectional view taken along the lines VI-VI of Figure 5, showing the annular cushion filled with the fluid.

**Figure 7** is an illustration of the device of Figure 5 shown during systole where the pressure wave in the blood has opened up the inner wall of the cushion forcing liquid into the reservoir which expands to accommodate the fluid.

**Figure 8** is a transverse sectional view taken along the lines VIII-VIII of Figure 7.

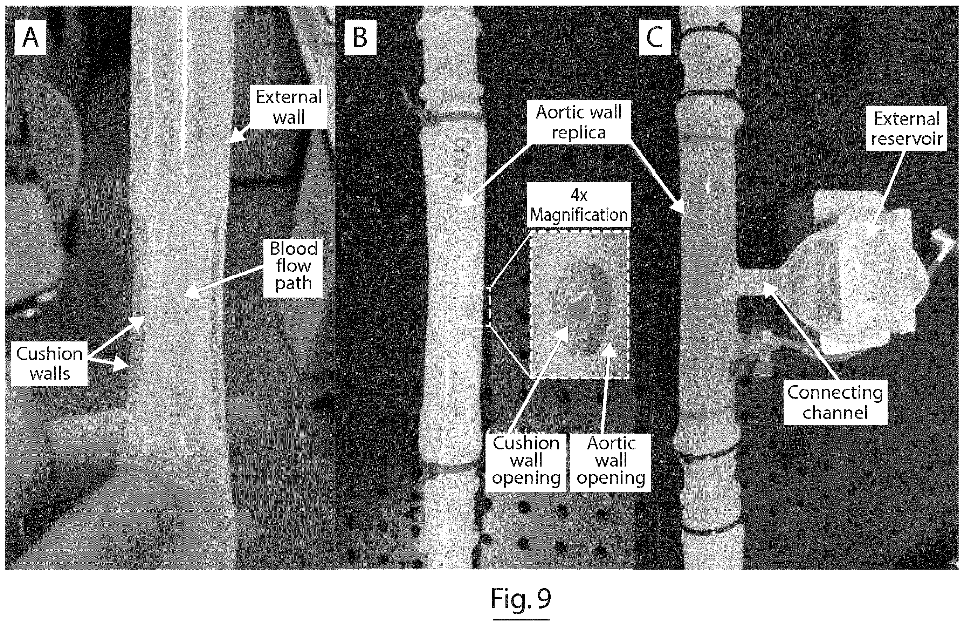

**Figure 9:** Device prototype I with active cushion and passive reservoir (A), Simulated use of device with openings of the cushion and artificial aortic wall (B) and Prototype under bench testing, including the attached external passive reservoir.

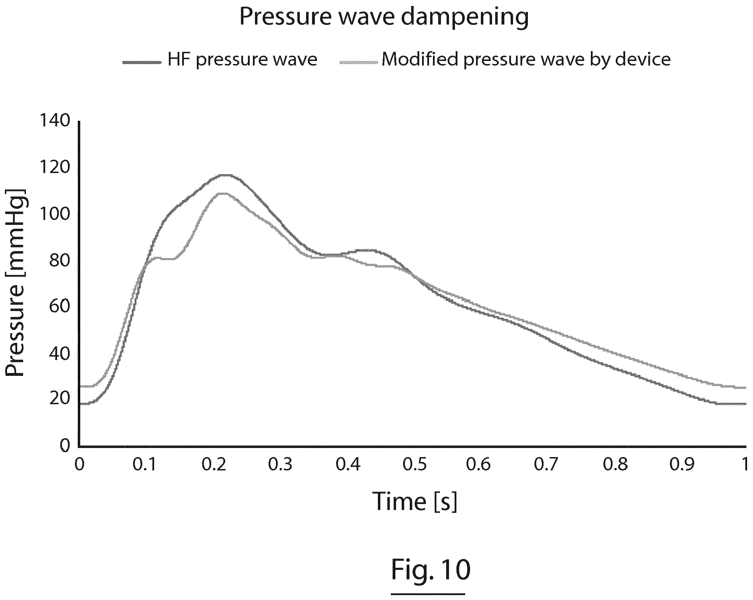

**Figure 10:** Heart failure replicated pressure waves (blue curve) versus dampened pressure wave by prototype (orange curve).

**Figure 11:** Device prototype II with passive cushion and active reservoir (C); Inner tube of cushion shown in a folded configuration (A, B).

## Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, age, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s) (for example, the reduction in accumulation of pathological levels of lysosomal enzymes). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an *effective amount* or a *therapeutically effective amount* of an agent defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure. Improvement may be observed in biological / molecular markers, clinical or observational improvements. In a preferred embodiment, the methods of the invention are applicable to humans, large racing animals (horses, camels, dogs), and domestic companion animals (cats and dogs).

In the context of treatment and effective amounts as defined above, the term *subject* (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, camels, bison, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human. As used herein, the term "equine" refers to mammals of the family *Equidae,* which includes horses, donkeys, asses, kiang and zebra.

The device of the invention is an implantable, in-dwelling, device. It may be configured for implantation for a short or long period of time. In one embodiment, the device is implanted and left in-situ for 1-52 weeks. During this time, the device will assist in reducing left ventricular afterload, thereby reducing load on the heart and help avoid the risk of heart failure in the subject. The device comprises three components, a cushion configured for holding a fluid, a reservoir configured to hold fluid, and a conduit that fluidically couples the cushion and reservoir. The cushion is generally annular with a central conduit to allow blood flow through the deployed device. The cushion may be compliant (i.e. resiliently deformable), so that pressure waves in the blood passing through the centre of the cushion expand the inner wall of the cushion (compressing the cushion) and pushing fluid in the cushion into the reservoir through the conduit. This has the effect of dampening pressure or velocity of the pressure pulse wave in the blood. When the pressure wave has passed, the cushion returns to a relaxed configuration, pulling fluid from the reservoir back into the cushion. This type of cushion is referred to herein as a "active" cushion. Alternatively, the cushion may be passive (i.e. non-compliant), and the reservoir may be compliant and resiliently deform when a pressure wave in the blood forces fluid in the cushion into the reservoir. The reservoir is generally implanted into the body adjacent the target blood vessel, for example 5-15mm away from the target blood vessel. In one embodiment, the reservoir is implanted subcutaneously. The conduit may include an adjustable valve to limit fluid transfer between the cushion and the reservoir. The valve may be configured for wireless operation. The conduit may include a sensor to detect fluid pressure in the conduit that is optionally configured for wireless relay of data. In one embodiment, the invention provides a system comprising a device of the invention and a processor configured to receive pressure data from the sensor and actuate the valve in response to pressure data received from the sensor. In one embodiment, the system comprises an external reservoir comprising fluid, a pump, and a conduit providing fluidic coupling between the device and external reservoir. In one embodiment, the processor is operatively coupled to the pump and configured to actuate the pump to modulate fluid level in the device in response to pressure signals received from the pressure sensor.

As used herein, the term "left ventricular afterload" refers to the workload on the left side of the heart. In most patients, the aorta can become stiffened, losing its ability to dampen the pressure wave generated each time the left ventricle contracts. Instead the pressure wave moves quickly down the aorta and is partially reflected towards the heart at a faster rate than normal. This early reflected waveform returns to the heart when the ventricle is still ejecting blood, significantly increasing the workload on the heart (left ventricle afterload).From a physiological standpoint, the stiffer the arterial system, the faster the reflected pressure wave returns from the periphery to the proximal aorta, adding earlier to the forward wave and eventually reaching the heart at systole instead of diastole, thus causing (1) augmentation of central systolic blood pressure increasing cardiac loading, and (2) reduction of central diastolic blood pressure decreasing coronary perfusion. An increase in arterial stiffness also increases the load on the heart, since it has to perform more work to maintain the stroke volume. Over time, this increased workload causes left ventricular hypertrophy and left ventricular remodelling, which can lead to heart failure. Several studies have proved this link. Hence strategies that reduce aortic stiffness can be the future of medical devices that are utilised in heart failure therapy.

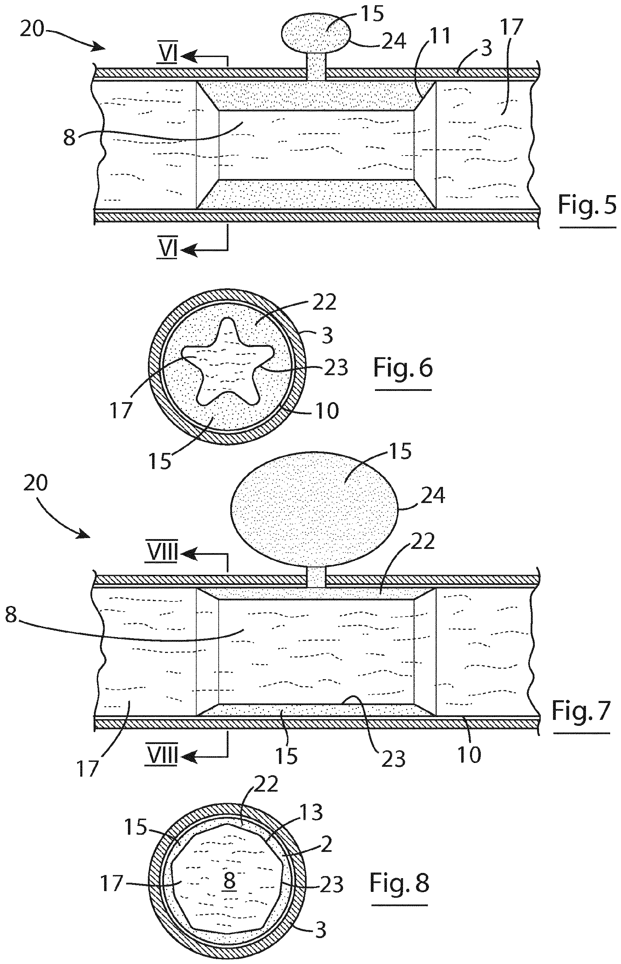

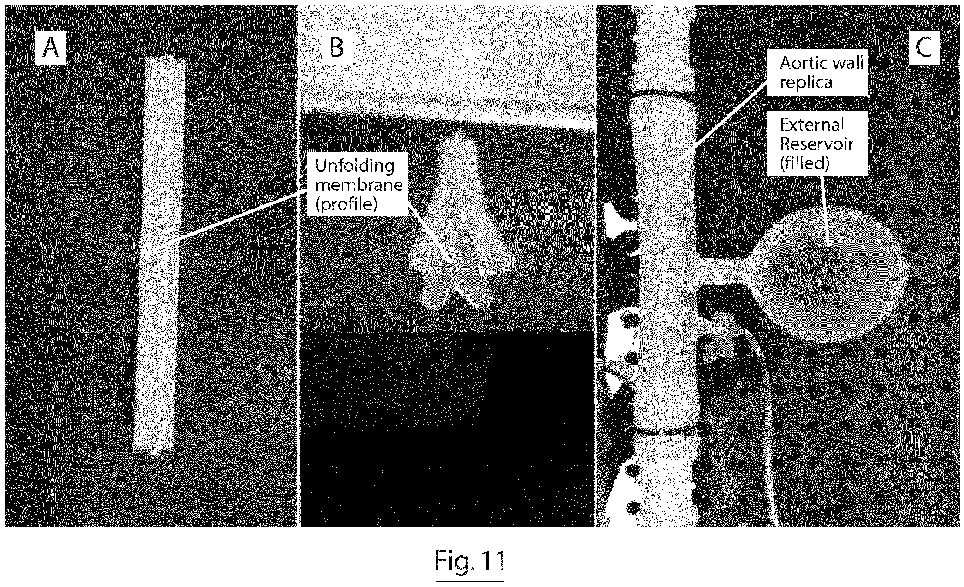

As used herein, the term "cushion" refers to a body configured to hold fluid and contract during systole and expand during diastole to dampen pressure waves in the blood. The cushion is configured to be disposed in a lumen of a section of vasculature, typically the arterial system, and ideally in the aorta. The cushion generally has an annular shape with a central lumen and is generally configured to abut the wall of the vasculature such that blood flows through the central lumen and not between the cushion and the surrounding walls of the vasculature. The cushion may be a compliant body that contracts during systole pushing blood in the reservoir and expands when the pressure wave in the blood has passed as the cushion returns to its original shape. This embodiment, in which the cushion generally comprises a resiliently deformable material, is referred to herein as an "active cushion". As the cushion is annular, the pressure wave in the blood exerts forces on the circumferential inner wall of the balloon, casing the inner circumferential wall to radially expand and the cushion to contract. In this embodiment, the reservoir is generally non-compliant and typically comprises a collapsible or foldable body. In this embodiment, the compliant cushion is generally primed with fluid prior to systole. In another embodiment, the cushion is not a compliant body, i.e. it can be a collapsible or foldable body, and the reservoir is generally a compliant body. In this embodiment the cushion is generally referred to as a "passive cushion". The cushion may have an inner circumferential wall that adjustable from a folded configuration (i.e. star shaped - Fig. 6 and Fig.11A and B) prior to systole, to an unfolded configuration during systole (Fig. 8). Examples of foldable cushions are described in more detail below. In one embodiment, the cushion has a length of 50-300mm. In one embodiment, the cushion has a central fluid receiving waist section, and end sections without a fluid receiving cavity. In one embodiment, the waist section has a length of about 50-200mm. In one embodiment the end sections have a length of about 10-50mm each. The inner diameter of the cushion is typically in the range of 5 to 35mm. The outer diameter is typically in the range of 15 to 40mm. The wall thickness of the cushion walls is typically in the range of 0.1 to 0.5mm. The cross-section of the annular cushion is generally configured to match the cross-section of the target vessel into which the device is to be implanted, and may be circular, oval-shaped, elliptical or have a regular or irregular polygonal cross-sectional shape. The cushion may be straight or curved, or tapered along all or part of its length. The cushion (or at least part of the cushion) is generally formed form a hyper-elastic elastomer with viscoelastic properties.

As used herein, the term "compliant" as applied to the cushion or reservoir should be understood to mean that the cushion or reservoir comprises a fluid receiving resiliently deformable body, that can expand from a relaxed configuration to an expanded configuration upon receipt of pressurised fluid.

Typically, the cushion or reservoir has a compliance in the range: 5 to 200 x $10^{-4}$/mmHg, based on formula (1) below.

$$C = \frac{1}{A_{sys}} \cdot \frac{(A_{sys} - A_{dia})}{(P_{sys} - P_{dia})} \text{ (Vorp et al. 1996)} \qquad (1)$$

with $A_{sys}$ - cross sectional area at systolic pressure, $A_{dia}$ - cross sectional area at diastolic pressure, $P_{sys}$ - systolic pressure, $P_{dia}$ - diastolic pressure. With this formula C = 72.4 x $10^{-4}$/mmHg.

[Vorp DA, Mandarino WA, Webster MW, et al. Potential influence of intraluminal thrombus on abdominal aortic aneurysm as assessed by a new non-invasive method. Cardiovasc Surg. 1996; 4(6): p. 732-739.]

As used herein, the term "target vessel" refers to a section of the vascular system, for example a section of an artery or vein. In one embodiment, the device is configured for use in the arterial system, in particular the aorta. In one embodiment, the device is positioned (or configured for positioning) in a mid-point of the descending thoracic aorta distal (for example at least 2 cm distal) of the left subclavian artery branch and proximal of the celiac artery branch. In another embodiment, the device is positioned (or configured for positioning) in a distal section of the descending thoracic aorta, distal of the renal arteries. In another embodiment, the device is positioned (or configured for positioning) in an ascending section of the aortic arch, proximal of the brachiocephalic artery.

As used herein, the term "foldable" as applied to the cushion or reservoir generally means that the cushion or reservoir is configured to passively fold as fluid is pulled from the cushion or reservoir and passively unfold as fluid is pushed into the cushion or reservoir.

The walls can be formed from a graft, textile woven/knitted/braided fabric or hyperelastic elastomer with viscoelastic properties. The textile fabric could be comprised of a monofilament or multifilament yarn. Thus, the walls can be configured to (1) expand first then unfold and expand after unfolding, (2) unfold first and then expand (3), or only unfold with no expansion. In embodiments where the cushion has a waist section, the waist section can comprise a biological material.

As used herein, the term "reservoir" refers to a body configured to receive fluid from the cushion during systole. The reservoir may be passive (i.e. it receives fluid passively during systole and allows most of the fluid to return to the cushion passively during diastole). Generally, in this embodiment, the reservoir is a collapsible or foldable structure that changes in volume due to fluid movement to and from the cushion, and not due to any resilient deformability of the reservoir. Generally, the reservoir has a volume at least three times the volume of the cushion. Generally, in this embodiment, the reservoir contains some fluid prior to systole. In another embodiment, the reservoir may be active, i.e. it may be a resiliently deformable (or compliant) body, that deforms under pressure during systole as fluid is transferred from the cushion to the reservoir. Generally, in this embodiment, the reservoir is primed with fluid prior to systole. The device is generally configured for the reservoir to be positioned outside the vasculature, generally adjacent the section of vasculature where the cushion is implanted. Generally, the conduit extends from an outer circumferential wall of the cushion, i.e. approximately radially outwardly of the cushion. In another embodiment, the device is configured for implantation of the reservoir in the vasculature, generally close to or adjacent the cushion. In this embodiment, the conduit may extend from an end of the cushion.

As used herein, the term "fluid" refers to a liquid or a gas. Preferably, the fluid is a liquid, ideally a substantially non-compressible liquid such as saline. The liquid may contain solid deformable particles, or gas bubbles.

As used herein, the term "conduit" refers to a conduit extending between and fluidically coupling the cushion and the reservoir. Typically, the conduit has a bore that is sufficiently large to avoid significant pressure increases in the fluid during transfer between the cushion and reservoir. For example, the conduit may have a bore of from 3 and 50 mm. Generally, the conduit is short, for example from 3 to 15 mm. Generally, a shorter conduit is preferred. The conduit may have a wall thickness of 0.1 to 0.5mm. The conduit may be formed from a hyperelastic elastomer with viscoelastic properties. Typically, such material is part of the following categories: natural rubbers, silicone rubbers and polyurethane rubbers. The conduit generally extends radially outwardly from a circumferential periphery of the cushion. In one embodiment, the conduit is orthogonal to a longitudinal axis of the cushion. In use, the conduit may be configured to pass through a hole in the vasculature, and the hole is generally configured to be slightly smaller than the diameter of the conduit such that a fluid tight seal is established between the conduit and the hole when the conduit is threaded through the hole. The conduit may incorporate a valve configured to meter passage of fluid through the conduit. The valve may incorporate an actuation means configured for remote actuation (for example RF-controlled valve). In another embodiment, the actuation means may include a pressure sensor configured to detect pressure in the fluid in the conduit, and a processor configured to actuate the valve in response to pressure signals received from the sensor. The device may be configured so that the transvascular conduit can be retrofitted to the cushion and/or the reservoir. In one embodiment, where the reservoir is implanted in the vasculature, the conduit may extend from an end of the cushion.

As used here, the term "annular cross section" or "annular" as applied to the cushion means that the cushion has a central lumen for blood flow. The outer circumference of the cushion is generally configured to abut the inner circumference of the vasculature into which is inserted, and is usually round, and typically has an inner circumference that may also be round. Generally, the cushion is configured such that the central lumen has a cross-sectional area that is at least 10% and preferably up to 90% (for example 10% to 90%, 2% to 90%, 30% to 90, 40% to 90%, 50%-90%, 60% to 90%, 70% to 90%, or 80%-90%) of the cross-sectional area of the vasculature, along at least 50% of the length of the cushion, prior to systole.

As used herein, the term "anchoring element" refers to an element that is associated with one end of the cushion and is generally adjustable from a radially contracted configuration to a radially expanded configuration, whereby when the cushion is disposed within the lumen of the vasculature the expanded anchoring element urges the end of the cushion into circumferential contact with the vasculature to prevent blood flow between the annular cushion and the vasculature. The anchoring element ensures a fluid tight seal between one end of the cushion and the vasculature to ensure that all blood flow is through the central lumen of the vasculature. Generally, the cushion comprises two anchoring elements, one associated with each end of the cushion. Generally, the anchoring element is coupled to an inner circumference of the cushion. Generally, the anchoring element is a radially expansible structure, for example a stent. In one embodiment, the cushion comprises an elongated body with distal and proximal ends and a cushion section disposed between the ends. In one embodiment, an anchoring element is coupled to the distal and/or proximal ends of the cushion.

As used herein, the term "fluid" maybe a liquid or a gas, or a mixture of liquid and gas. The fluid may contain solid deformable particles. In one embodiment, the fluid is a liquid containing gas bubbles. Generally, the liquid is saline.

The device of the invention may be employed to treat various conditions related to the vascular system, including cardiovascular conditions, hypertensive disorders, (peripheral) vascular disorders, complications associated with EVAR ((endovascular aneurysm repair). and renal disease. In one embodiment, the treatment is a symptomatic therapy. In one embodiment, the treatment is a causative therapy. In one embodiment, the cardiovascular disease is selected from heart failure, atrial fibrillation, stroke, and coronary artery disease. In one embodiment, the hypertensive disorder is selected from hypertension.

Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

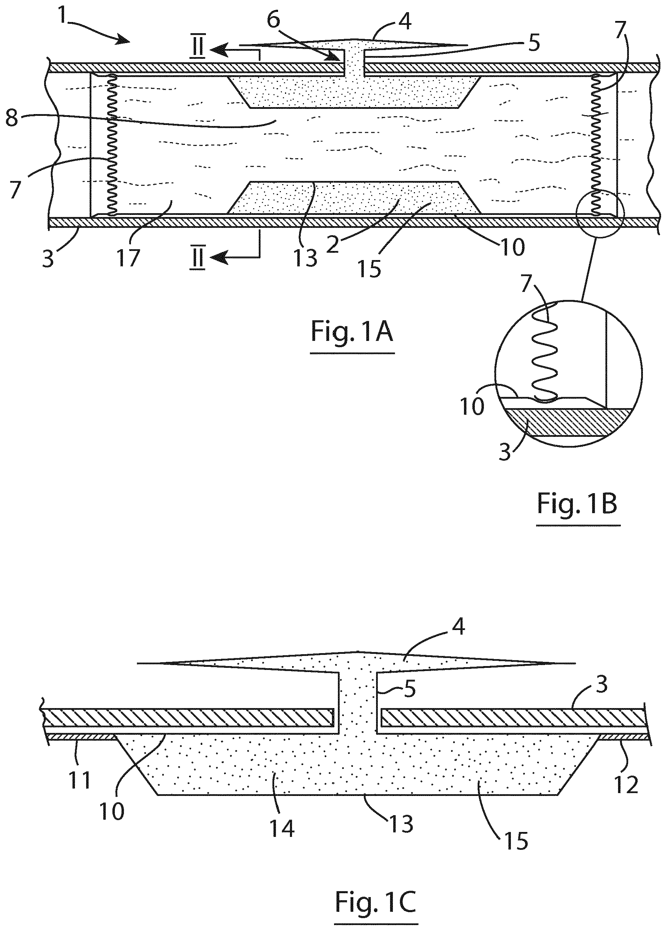

Referring to the drawings, and initially to Figures 1 to 2, a device according to a first embodiment of the invention is illustrated, indicated generally by the reference numeral 1. the device 1 comprises a compliant cushion body 2 disposed within a section of an aorta 3, a passive reservoir 4 disposed adjacent to the aorta, and a transvascular conduit 5 coupling and fluidically connecting the cushion body 2 and reservoir 4 via an aperture 6 in the aorta. The cushion body 2 includes two anchoring elements 7 that serve to anchor the cushion body in the aorta and circumferentially seal each end of the cushion body against the inner wall of the aorta, and a central lumen 8 for passage of blood. The device is illustrated prior to systole, when the compliant cushion is in a relaxed configuration.

In more detail, the cushion body comprises an outer tube 10 made of a hyperelastic elastomer with viscoelastic properties (examples include natural rubbers, silicone rubbers and polyurethane rubbers) configured to fit snugly within the lumen of the vasculature, an inner compliant tube 11 made of a hyperelastic elastomer with viscoelastic properties (examples include natural rubbers, silicone rubbers and polyurethane rubbers) having end sections 12 attached to an inner wall of the outer tube and a compliant waist section 13 disposed between the end sections that together with an inner wall of the outer tube defines an annular lumen 14 that contains a fluid 15 and is in fluid communication with reservoir 4 via the transvascular conduit 5. The waist section generally is formed from a hyperelastic elastomer with viscoelastic properties. The inner tube 11 defines the central lumen 8 for flow of blood 17.

In more detail, the anchoring elements 7 comprises annular stents that are coupled to the ends of the inner tubes 11 and adjustable from a radially contracted delivery configuration (not shown), to a radially expanded anchoring configuration (Fig. 1B) where the stents force the inner tube 11 into circumferential engagement with an inner wall of the aorta 3, sealing the ends of the cushion body to aorta to prevent any ingress of blood between the cushion body and the aorta, and ensuring that all blood is channelled through the central lumen 8 of the cushion body. The stents are configured to maintain their radially expanded configuration once expended. Examples of anchoring stents that may be employed as part of the device of the invention include wire coil Z-stents or/and laser cut stents of various architectures. In use, the stents may be expended by placing a balloon catheter within the lumen of the cushion and expanding the balloon to force the stents into the expanded configuration.

The passive reservoir 4 comprises a foldable body that is shown in a folded, and substantially flat, configuration in Figure 1. This is shown prior to the arrival of a pressure wave during (systole) where the device is configured such that all or most of the fluid 15 in the device is disposed in the compliant cushion, and the reservoir 4 is not primed with fluid.

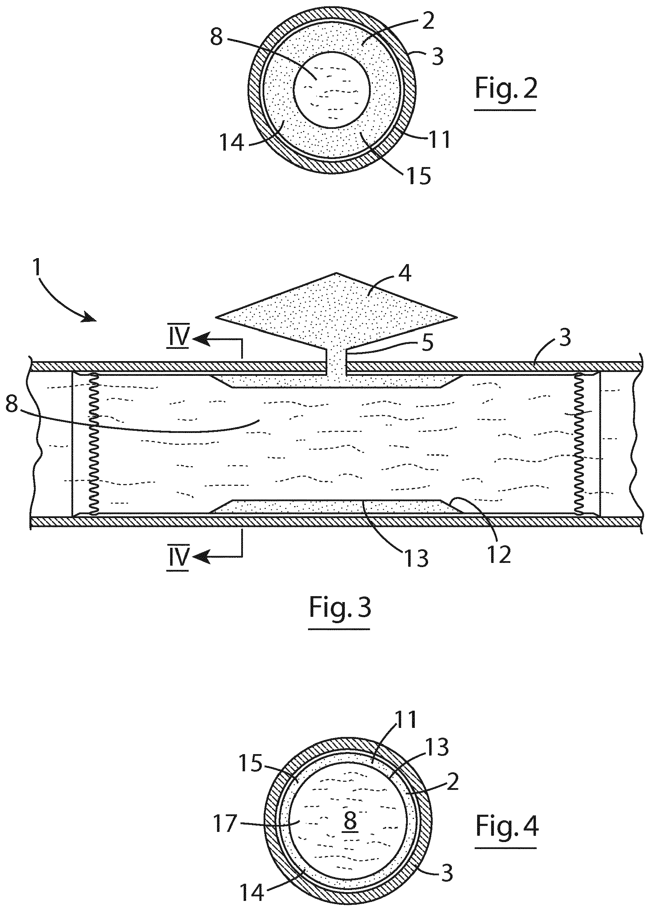

Figures 3 and 4 illustrates the device of Figures 1 and 2 during systole, where the arrival of a pressure wave in the blood and the resultant increase in blood pressure in the aorta pressurises the cushion causing the compliant waist section 13 of the inner tube 11 to expand radially causing the annular cushion to contact and force the fluid 15 from the cushion body through the transvascular conduit 5 and into the passive reservoir 4, causing the reservoir to unfold. The change in shape in the compliant cushion 2 is best illustrated by comparing Figures 2 and 4, which can be seen that the increased pressure in the blood causes the compliant waist section 13 to radially expand, which in turn causes the cushion to contract thereby dampening the pressure wave in the blood. Once the cushion has contracted, it is no longer in a relaxed configuration, but is biased to return to the relaxed configuration due to the compliant waist section 13 once the pressure wave in the blood has passed. Once the blood pressure in the central lumen reduces during diastole, the compliant waist section 13 returns to it non-expanded configuration, increasing the volume in the compliant cushion, and pulling fluid 15 from the reservoir back into the cushion via the transvascular conduit 5. It is important to note that in this embodiment (active cushion/passive reservoir), the reservoir 4 plays a passive role in the transfer of fluid between the cushion 2 and reservoir 4.

Referring now to Figures 5 to 8, a device according to a second embodiment of the invention is illustrated, indicated generally by the reference numeral 20, in which parts identified with reference to the previous embodiments are assigned the same reference numerals. In this embodiment, the device comprises an active reservoir having a compliant fluid-receiving body 24, and a passive cushion 22 having an inner tube 11 with a waist section 23 that is fluted longitudinally and adjustable from a folded configuration (Figure 6) to an unfolded, opened-up, configuration (Figure 8). As illustrated in Figure 5, the active reservoir is primed with fluid 15 prior to systole. In Figures 5 and 6, the device is shown prior to systole, with the cushion in a folded configuration, and a star-shaped central lumen 8 providing a passage for blood 17. In this configuration, the compliant reservoir is in a relaxed configuration in Figure 5 and 6, with the reservoir primed with fluid. In Figures 7 and 8, the device is shown during systole, where a pressure wave in the blood has increased the blood pressure in the aorta, forcing the cushion to unfold and decrease in volume, forcing liquid into the compliant reservoir which expands, and resulting in a dampening in the pressure wave. Due to the resiliently deformable nature of the compliant reservoir, once the pressure wave has passed the fluid is pushed by the compliant reservoir back into the cushion, which increases in volume passively due to the waist section 23 returning its folded configuration shown in Figure 6.

Implantation

In use, the device is implanted into a subject with the cushion implanted into a target section of the vasculature (in this case the aorta), the reservoir implanted into the chest cavity close to the target section of the vasculature, and the transvascular conduit threaded through a hole formed in the aorta. The device may be implanted transluminally, surgically, or by a combination of transluminal and surgical procedures.

Surgical Implantation

The device may be implanted surgically. In this embodiment, the chest is opened, and a target section of the vasculature is surgically resected, and a small hole made in the wall of resected section of the vasculature. The cushion sleeve of the device of the invention is then inserted into the resected section of the vasculature. The cushion sleeve includes a circumferential hole configured to receive on end of the transvascular conduit. The position of the implanted cushion sleeve in the resected section of the vasculature is then adjusted until the hole in the cushion aligns with the small hole in the resected section of vasculature. One end of the transvascular conduit is then inserted into the small hole in the vasculature and coupled with the hole in the cushion. The anchoring stents disposed on the inside wall of the cushion sleeve are then deployed, to anchor the cushion sleeve in position and fluidically seal the ends of the cushion sleeve to the inner wall of the resected section of vasculature. Once anchored in place, the opposite end of the transvascular conduit may then fluidically coupled to the reservoir, and the device can be primed with fluid.

Transluminal Implantation

The device may be implanted transluminally. In this embodiment, access is gained either by percutaneous access or by open incisions surgically to the left and right external iliac arteries, in the groin, and guidewires are inserted and navigated along the aorta artery, in parallel, in a retrograde fashion; Seldinger technique. The cushion is collapsed and mounted in a delivery system, which is inserted over the left guidewire, and guided with the use real time fluroscopy, until it reaches the destination site in the aorta. The optimum destination site in the aorta is the ascending thoracic aorta to achieve the maximal target goal. However anatomically it may not be the best implantation site. An optimum balance would be in the descending thoracic aorta distal to the left Subclavian artery origin without compromising target goals. The device can be technically implanted in other areas of the aortic tree, but this may compromise on target goals with the current version. The cushion is deployed, and a balloon is used to expand the proximal stent for anchoring. The delivery system is then retracted, leaving the original guidewire in place. Through the cushion distal end, there is a second guidewire, which was inserted during the mounting phase of the cushion into the delivery system. A second delivery system, carrying a needle, is inserted from the groin, and advanced over the second guidewire, under fluoroscopic guidance, until it enters the cushion. When it reached the mid length of the cushion, the needle is advanced to perforate the cushion wall and the aortic wall orthogonally. Then, the tip of the system, carrying the needle, is advanced, following the needle path, to expand the hole in the aortic wall. The needle is retracted, and the guidewire is advanced through the hole. The needle and its delivery system are retracted and a third delivery system carrying the reservoir, in a collapsed state, is advanced over the same guidewire, through the cushion and the hole in the aortic wall for placement. A stent is then deployed in the overlap region of the cushion and reservoir area to keep the aortic wall hole open, and to form the conduit. The delivery system is retracted, and the second guidewire will remain in place, to allow for a microcatheter to be placed in the cushion for the fluid filling phase. A plug is pulled when the microcatheter is retracted with the guidewire from the cushion wall. A balloon is advanced over the main guidewire and expanded at the distal end of the cushion to expand the stent and anchor the device. It is anticipated that modifications will be made to the device to negate the requirement of 'the reservoir' hence making the implantation technique of the device to a minimally invasive percutaneous implantation technique.

Hybrid implantation

With this approach the device main body, that holds the cushion, is delivered following the steps described for the transluminal implantation, while the reservoir is delivered following the surgical approach steps. After the device body is positioned in the designated aorta region, the aortic wall and the cushion external wall are punctured via the surgical approach and then the proximal end of the conduit, is positioned and attached to the cushion. The sealing to the aortic and cushion walls is achieved by deploying a stent scaffold inside the proximal half of the conduit. Then the reservoir is connected to the distal end of the conduit via custom-made end to end connectors.

Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

**Claims**

1. A device (1, 20) to reduce left ventricle afterload in a target blood vessel in a mammal, comprising:

   a cushion (2, 22) configured for positioning within a lumen of the target blood vessel (3) and receipt of a fluid (15) and configured to contract during systole and expand during diastole;
   a reservoir (4, 24) having a cavity for receiving the fluid (15); and
   a conduit (5) extending between and fluidically coupling the cushion and the reservoir, whereby during use fluid in the cushion is transferred to the reservoir during systole and returned to the cushion during diastole,

   **characterised in that** the cushion (2, 22) has an annular cross section defining a central lumen (8) for blood flow and is configured for positioning in the lumen of the target vessel abutting an inner wall of the target vessel, whereby during use blood flow is directed through the central lumen of the cushion.

2. A device according to Claim 1, in which the cushion is a compliant cushion (2) configured for compression by a pressure wave in blood (17) passing through the central lumen (8) of the cushion during systole to push fluid to the reservoir (4) and decompression during diastole to pull fluid back into the cushion from the reservoir, and in which the reservoir is configured to passively accept fluid during compression of the compliant cushion and passively discharge fluid during decompression of the compliant cushion.

3. A device according to any preceding Claim, including an anchoring element (7) associated with at least one end of the cushion (2, 22) and adjustable from a radially contracted configuration to a radially expanded configuration, whereby when the cushion is disposed within the lumen of the vasculature (3), the expanded anchoring element urges the end of the cushion into circumferential contact with the vasculature to prevent blood flow between the annular cushion and the vasculature.

4. A device according the Claim 3, in which the anchoring element is a radially expandable stent associated with an inner circumference of a proximal end of the cushion.

5. A device according to any preceding Claim, in which the conduit (5) has a length from 3mm to 15mm.

6. A device according to any preceding Claim, in which the conduit (5) extends radially outwardly of the cushion.

7. A device according to Claim 6, in which the reservoir is configured for implantation into a body cavity adjacent the target blood vessel.

8. A device according to any of Claims 2 to 7, in which the complaint cushion comprises:

   an outer tube (10) configured to fit snugly within the lumen of the target blood vessel,
   an inner tube (11) having end sections (12) attached to an inner wall of the outer tube and a compliant waist section (13) disposed between the end sections, and
   a fluid receiving annular lumen defined by the outer tube and the central waist section.

9. A device according to Claim 8, in which the outer tube, inner tube and compliant waist section are formed from a hyper-elastic elastomer with viscoelastic properties.

10. A device according to any preceding Claim, in which the reservoir for fluid comprises a foldable structure (4) configured for adjustment from an initial folded configuration to an unfolded, fluid holding, configuration.

11. A device according to any preceding Claim, configured such that some fluid is disposed within the reservoir prior to systole.

12. A device according to any preceding Claim, in which the conduit (5) comprises a valve and a valve actuation mechanism including remote actuation means.

13. A device according to any of Claims 1 and 3 to 12, in which the reservoir for fluid comprises a compliant fluid-receiving body (24) and the cushion (22) is configured to passively accept fluid during diastole.

EP 3 815 723 A1

**14.** A device according to any preceding Claim, in which the reservoir (4, 24) comprises an injection port fluidically coupled to the fluid receiving cavity.

**15.** A device according to any preceding Claim, comprising a plurality of reservoirs fluidically coupled to the cushion in parallel or in series.

13

Fig. 1A

Fig. 1B

Fig. 1C

Fig. 2

Fig. 3

Fig. 4

Fig. 5

Fig. 6

Fig. 7

Fig. 8

Fig.9

## Pressure wave dampening

— HF pressure wave     — Modified pressure wave by device

Fig. 10

Fig. 11

Europäisches
Patentamt

European
Patent Office

Office européen
des brevets

# EUROPEAN SEARCH REPORT

Application Number

EP 19 20 6637

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | US 2011/124951 A1 (WALSH PETER WILLIAM [AU]) 26 May 2011 (2011-05-26)<br>* the whole document *<br>* paragraphs [0019] - [0038] *<br>* "Discrete Energy Storage System-Windkessel"; "Intraluminal Balloon Cuff 1200";<br>paragraphs [0108] - [0127], [0134]; figures 1,4 * | 1,3-7,<br>11-14<br>2,8-10,<br>15 | INV.<br>A61M1/10<br>A61M1/12 |
| X | US 6 030 336 A (FRANCHI PIERRE [FR])<br>29 February 2000 (2000-02-29)<br>* figures 1,2 * | 1,3,4,11 | |
| X | US 2010/197994 A1 (MEHMANESH HORMOZ [IR])<br>5 August 2010 (2010-08-05)<br>* figure 1 * | 1,3,4,11 | |
| X | US 2006/178731 A1 (TOWER ALLEN J [US])<br>10 August 2006 (2006-08-10)<br>* paragraphs [0019] - [0024]; figures 2,3 * | 1,3,4,11 | |
| X | WO 00/35515 A1 (CORVASCULAR INC [US]; PASPA PAUL [US]; NAGAO REX [US])<br>22 June 2000 (2000-06-22)<br>* figure 1 * | 1,3,4,11 | TECHNICAL FIELDS SEARCHED (IPC)<br><br>A61M |
| A | US 2017/007754 A1 (BABBS CHARLES F [US] ET AL) 12 January 2017 (2017-01-12)<br>* the whole document *<br>* figures 1,4-6A * | 1 | |

The present search report has been drawn up for all claims

| Place of search | Date of completion of the search | Examiner |
|---|---|---|
| The Hague | 10 April 2020 | Van Veen, Jennifer |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 20 6637

This annex lists the patent family members relating to the patent documents cited in the above-mentioned European search report.
The members are as contained in the European Patent Office EDP file on
The European Patent Office is in no way liable for these particulars which are merely given for the purpose of information.

10-04-2020

| Patent document cited in search report | | Publication date | Patent family member(s) | | | Publication date |
|---|---|---|---|---|---|---|
| US 2011124951 | A1 | 26-05-2011 | AU | 2005218677 | A1 | 15-09-2005 |
| | | | CA | 2599434 | A1 | 15-09-2005 |
| | | | EP | 1720584 | A1 | 15-11-2006 |
| | | | JP | 2007526039 | A | 13-09-2007 |
| | | | US | 2008194905 | A1 | 14-08-2008 |
| | | | US | 2011124951 | A1 | 26-05-2011 |
| | | | WO | 2005084730 | A1 | 15-09-2005 |
| US 6030336 | A | 29-02-2000 | AT | 277651 | T | 15-10-2004 |
| | | | DE | 69730995 | D1 | 04-11-2004 |
| | | | DE | 69730995 | T2 | 23-02-2006 |
| | | | EP | 0959912 | A1 | 01-12-1999 |
| | | | FR | 2744924 | A1 | 22-08-1997 |
| | | | JP | 2000513244 | A | 10-10-2000 |
| | | | PT | 959912 | E | 28-02-2005 |
| | | | US | 6030336 | A | 29-02-2000 |
| | | | WO | 9730740 | A1 | 28-08-1997 |
| US 2010197994 | A1 | 05-08-2010 | EP | 2016961 | A1 | 21-01-2009 |
| | | | US | 2010197994 | A1 | 05-08-2010 |
| | | | WO | 2009010302 | A2 | 22-01-2009 |
| US 2006178731 | A1 | 10-08-2006 | US | 2006178731 | A1 | 10-08-2006 |
| | | | WO | 2006086048 | A1 | 17-08-2006 |
| WO 0035515 | A1 | 22-06-2000 | AU | 2188700 | A | 03-07-2000 |
| | | | WO | 0035515 | A1 | 22-06-2000 |
| US 2017007754 | A1 | 12-01-2017 | EP | 3115071 | A1 | 11-01-2017 |
| | | | US | 2017007754 | A1 | 12-01-2017 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9017359 B

- US 9333328 B

**Non-patent literature cited in the description**

- **VORP DA ; MANDARINO WA ; WEBSTER MW et al.** Potential influence of intraluminal thrombus on abdominal aortic aneurysm as assessed by a new non-invasive method. *Cardiovasc Surg.,* 1996, vol. 4 (6), 732-739